# EUROPEAN PATENT SPECIFICATION

(11) **EP 0 371 821 B1**
(45) Date of publication and mention of the grant of the patent: **25.05.1994**
(21) Application number: 89312526.0
(22) Date of filing: 30.11.1989
(51) Int. Cl.: C07C 213/02, C07C 219/08

(54) **A process for the production of substituted acryloyloxyhydroxypropyltrialkylammonium chloride**
Verfahren zur Herstellung von substituiertem Acryloyloxyhydroxypropyltrialkylammoniumchlorid
Procédé de préparation de chlorure d'acryloyloxy-hydroxypropyl trialkyl-ammonium substitué

(30) Priority: 30.11.1988 US 278297; 30.11.1988 US 278299; 30.11.1988 US 278300
(43) Date of publication of application: 06.06.1990
(73) Proprietor: RHONE-POULENC SPECIALTY CHEMICALS CO., New Jersey 08512-7500 (US)
(72) Inventor: Chiang, William Gong-Jean, Fayetteville, NY 13066 (US); Jobbins, Richard McIntyre, Marcellus, NY 13108 (US); Popule, Michael Paul, Syracuse, NY 13204 (US)
(74) Representative: Brock, Peter William

(56) References cited:
- DE-A- 1 593 421
- US-A- 4 169 208
- CHEMICAL ABSTRACTS, vol. 81, no. 26, 30th December 1974, page 28, abstract no. 170237x, Colombus, Ohio, US
- CHEMICAL ABSTRACTS, vol. 102, no. 20, page 14, abstract no. 167349g, Columbus, Ohio, US
- CHEMICAL ABSTRACTS, vol. 79, no. 7, 20th August 1973, page 316, abstract no. 41973u, Columbus, Ohio, US; & JP-A-73 34 116

## Description

The preparation of 3-(R)acryloyloxy-2-hydroxypropyltrialkylammonium halide monomer is enhanced by carrying out the reaction in a media which is a non-solvent for the monomer, but is a solvent for the reactants.

The present invention relates to a process for making 3-substituted acryloyloxy-2-hydroxypropyltrialkyl ammonium chloride and its isomer, 2-substituted acryloyloxy-3-hydroxypropyltrialkylammonium chloride, wherein the substituent is hydrogen or lower alkyl of 1 to 4 carbon atoms.

The quaternary salt 3-methacryloyloxy-2-hydroxypropyltrimethylammonium chloride and its isomer 2-methacryloyloxy-3-hydroxypropyltrimethylammonium chloride are generally referred to as MAHTAC.

MAHTAC is a cationic vinyl monomer which can be polymerized or copolymerized with a number of other vinyl monomers to produce polymers which are useful in antistatic, flocculation or sludge dewatering applications. If high molecular weight polymers are to be synthesized without gellation due to crosslinking, it is essential that the MAHTAC purity with regards to molecules with two or more vinyl groups be very high. It is also essential that the yield be high for economic viability.

DE-A-1593421 describes the synthesis of MAHTAC by reacting aqueous glycidyltrimethylammonium chloride (GTAC) with methacrylic acid, or by reacting together epichlorohydrin, methacrylic acid and trimethylamine.

US-A-4169208 discloses the quaternization of beta(dialkylamino)ethyl acrylates and methacrylates with methyl chloride in the presence of acrylonitrile as solvent.

This invention provides a process for preparing a mixture of a 3-(R)acryloyloxy-2-hydroxypropyltrialkylammonium halide with its 2-(R)acryloyloxy-3-hydroxypropyltrialkylammonium halide isomer. According to the invention the halohydroxypropyl ester of an acid of the formula CH₂=C(R)COOH wherein R is hydrogen or alkyl of 1 to 4 carbon atoms is reacted with from 0.5 to 2.0 moles, per mole of halohydroxy propyl ester, of a trialkylamine at a temperature of 25 to 90°C in from 35 to 85 weight % of a polar aprotic liquid which is non-solvent for said 3-(R)acryloyloxy-2-hydroxypropyltrialkylammonium halide and its isomer and the molar ratio of said hydroxypropyl ester to trialkylamine is from 1.0:1.0 to 1.0:2.0.

According to one embodiment, the halohydroxypropyl ester is prepared by reacting methacrylic acid and epichlorohydrin in the presence of a mixture of 3-methacryloyloxy-2-hydroxypropyltrimethylammonium chloride monomer and its isomer, 2-methacryloyloxy-3-hydroxypropyltrimethylammonium chloride monomer as catalyst at a temperature and for a time sufficient to form said ester.

Additional aspects of this invention include the optional use of a catalyst having an epoxy functional group, the charging of reaction product during the reaction to seed the reaction and forming the hydroxypropyl ester starting reactant in a non-solvent for the hydroxypropyl ester.

The present invention provides a versatile process for the preparation of substituted acryloyloxy-2-hydroxypropyltrialkylammonium chloride (which can also be referred to as 3-(R)acryloyloxy-2-hydroxypropyltrialkylammonium chloride and its isomer 2-(R)acryloyloxy-3-hydroxypropyltrialkylammonium chloride. In the foregoing, the R group (or substituent) for the acryloyloxy is either hydrogen or alkyl having 1 to 4 carbon atoms. The other alkyl group also contains from 1 to 4 carbon atoms. For convenience, the invention will hereafter be referred to in terms of its most desirable product, namely 3-methacryloyloxy-2-hydroxypropyltrimethylammonium chloride and its isomer 2-methacryloyloxy-3-hydroxypropyltrimethylammonium chloride which will be hereinafter be collectively referred to as MAHTAC. It will be readily apparent to those skilled in the art that by the selection of other substitutents , falling within the scope stated above, a number of other similar cationic vinyl monomers can be prepared.

The process of the present invention permits the synthesis of high purity MAHTAC in high yield without requiring special purifications or other treatments. The advantages of this invention are (1) the direct production of crystalline MAHTAC by carrying the reaction out in a solvent for the reactants and a non-solvent for MAHTAC; (2) the synthesis of MAHTAC at a commercially viable rate using an appropriate catalyst; and (3) the production of crystalline monomer of sufficient size to allow easy separation from the reaction medium.

In the process of this invention, 3-chloro-2-hydroxypropylmethacrylate and its isomer 2-chloro-3-hydroxypropylmethacrylate (collectively called CHPM) are reacted with anhydrous trimethylamine in a polar, aprotic solvent which is a good solvent for the reactants but a non-solvent for MAHTAC. As the reaction proceeds, the MAHTAC precipitates as a white crystal. This crystal is easily separated from the reaction solvent which contains the impurities of the reaction.

Synthesis of the CHPM has been detailed in patents and in several journals. It is also available as Sipomer®CHPM from Alcolac, Inc. CHPM is made by reacting stoichiometric or nearly stoichiometric quantities of epichlorohydrin and methacrylic acid at temperatures from about 50 to 100°C in the presence of a polymerization inhibitor and a catalyst. Catalysts may include amines, quaternary ammonium salts or one of a number of other compounds. Literature references teach purification by extraction of residual methacrylic acid with sodium bicarbonate, followed by acid and water washes and distillation. As a consequence of side reactions, the CHPM contains, among other impurities, from 0.1 to about 2.0% of 2-hydroxypropane-1,3-dimethacrylate (HPDMA) and 3-chloropropane-1,2-dimethacrylate (CPDMA). Both of these diesters are poorly removed by the CHPM purification procedure. Since they contain two double bonds, they are polymerization crosslinking agents. It is essential that they be reduced to levels below about 10 parts-per-million in any MAHTAC made from CHPM if the MAHTAC is to used in the synthesis of linear, high molecular weight polymers. One advantage of the invention described herein is that CHPM purification steps are not required for the synthesis of MAHTAC with low diester levels.

In the process of this invention CHPM is made according to the literature references or is purchased. Additionally, CHPM is advantageously prepared by methacrylic acid (MAA). and epichlorohydrin (EPI) are reacted in the presence of a mixture of 3-methacryloyloxy-2-hydroxypropyltrimethylammonium chloride and its isomer as catalyst in step (a). Commercially available methacrylic acid and epichlorohydrin may be used in the present invention. Preferably, the molar ratio of methacrylic acid used to epichlorohydrin is about 0.5:1.0 to 1.0:0.5. If the ratio of epichlorohydrin to methacrylic acid used exceeds 1.0:0.5, polyepichlorohydrin forms. More preferably, the molar ratio of methacrylic acid to epichlorihydrin is 0.8:1.0 to 1.0:0.8. Most preferably, the molar ratio of methacrylic acid to epichlorohydrin is 0.9:1.0 to 1.0:1.0. The use of a slight excess of epichlorohydrin is most preferred.

The reaction of methacrylic acid and epichlorohydrin may optionally be carried out in the presence of a non-solvent.

It has been discovered that a mixture of 3-methacryloyloxy-2-hydroxypropyltrimethylammonium chloride monomer and its isomer is a catalyst with advantages over triethylamine (a catalyst used in the art) in the formation of the chlorohydrin esters in step (a) of the monomer preparation.

Preferably, the molar ratio of the mixture of 3-methacryloyloxy-2-hydroxypropyltrimethylammonium chloride monomer and its isomer to methacrylic acid is 0.01:1.0 to 0.2:1.0. More preferably, the molar ratio of the MAHTAC mixture to methacrylic acid is 0.01:1.0 to 0.1:1.0. Most preferably, the molar ratio of the MAHTAC mixture to methacrylic acid is 0.02:1.0 to 0.04:1.0.

The methacrylic acid and epichlorohydrin are reacted in the presence of the MAHTAC mixture of chloride monomer and its isomer at a temperature and for a time sufficient to form a reaction product. Preferably, the methacrylic acid and epichlorohydrin are reacted in the presence of the MAHTAC mixture at a temperature of 25 to 100°C. More preferably, the temperature is 50 to 100°C; most preferably, the temperature is 65 to 90°C. More preferably, the reaction time is 3 to 24 hours; most preferably, the reaction time is 5 to 7 hours. Reaction pressure should be atmospheric.

Preferably, the reaction occurs in the presence of a polymerization inhibitor. Preferred polymerization inhibitors are p-methoxyphenol, hydroquinone and phenothiazine; these materials are available in commercial quantities. The polymerization inhibitors may be used in conventional amounts.

Although not wishing to be bound by theory, it is believed that the reaction mechanism of the step (a) synthesis involves formation of an intermediate ionic complex [catalyst-acid] and then insertion of the epichlorohydrin epoxy ring into the complex [ionic complex-epichlorohydrin) via trans-opening of the epoxide ring.

Purification of the CHPM solution before the conversion to MAHTAC is unnecessary. It has been found that although the use of a purified CHPM solution produces a high purity 3-methacryloyloxy-2-hydroxypropyltrimethylammonium chloride monomer, the overall process yield is lower and the reaction time is much longer than when an unpurified CHPM solution is used. If desired, crude CHPM solution can be purified to remove any residual methacrylic acid, epichlorohydrin and other impurities. To do this, the crude CHPM solution is first extracted with a saturated aqueous NaHCO₃ until the methacrylic acid is removed followed by vacuum distillation to remove any residual epichlorohydrin and other residual impurities.

One mole of CHPM and from 0.5 to 2 moles of anhydrous trimethylamine are added to sufficient solvent to form a solution containing from 35 to 85% of the solvent. More preferably, the mole ratio of trimethylamine to CHPM is 1.0:1.0 to 1.2:1.0. The solvent is selected so that the reactants are readily soluble but the MAHTAC has a limited solubility. The solvent should also not enter into any reactions with the reactants or products. Polar protic solvents are unsatisfactory since they may lead to hydrolysis or transesterifications. Water and alcohols are not satisfactory for this reason. Preferred solvents include tetrahydrofuran, acetone, acetonitrile, propionitrile, butyronitrile, dimethylformamide, dimethylsulfoxide, methyl ethyl ketone, ethyl acetate, and 1,1,2-trichlorethene. More preferred solvents are acetonitrile, acetone, tetrahydrofuran, methyl ethyl ketone, ethyl acetate and 1,1,2-trichlorethene. The most preferred solvent is acetonitrile.

This solution is then allowed to react under its natural vapor pressure, or any convenient higher pressure, at a temperature of from 25 to 95°C. More preferably, the temperature is from 45 to 85°C and most preferably from 50 to 70°C. Preferably, the reaction time is from 2 to 48 hours and more preferably, the reaction time is from 3 to 25 hours. Most preferably, the reaction time is from 4 to 10 hours.

Preferablyl the reaction is carried out in the presence of a polymerization inhibitor such as p-methoxyphenol, phenothiazine or hydroquinone. These materials are available commercially and are used in conventional amounts.

During the course of the reaction, MAHTAC precipitates as a white crystal. When the reaction is complete, the crystalline mass is separated from the reaction mixture by a conventional means; such as filtration or centrifugation. We have found centrifugation to be particularly effective. If desired, washing of the crystals may be done in the filter or the centrifuge to reduce the amount of "mother liquor" on the crystal surfaces. This reduces the impurities further.

It has been found that the rate of formation of MAHTAC can be accelerated by the presence of a component with an epoxy functional group. Although not wishing to be bound by theory, MAHTAC may be formed by at least two mechanisms. One is the direct alkylation of trimethylamine by chlorine containing functional group of the CHPM molecule yielding a quaternary ammonium chloride. The second involves the formation of glycidylmethacrylate from CHPM which is then attacked by the nucleophilic trimethylamine. In the process, MAHTAC is formed and a new molecule of glycidylmethacrylate is generated from CHPM. This process is repeated over and over until the reactants are consumed or the reaction stopped. This reaction is much faster than the direct alkylation. The overall reaction follows the scheme: 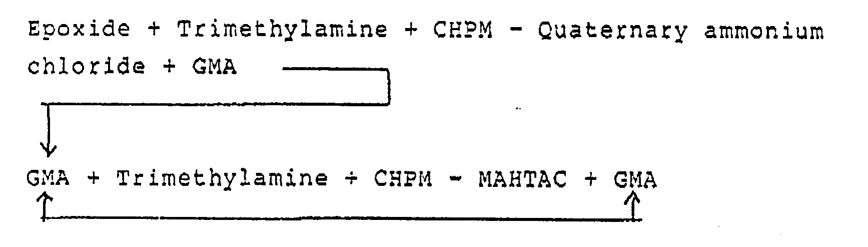
Many compounds with an epoxy functional group may react with trimethylamine to initiate this cycle. For example, a small amount of epichlorohydrin nay react with the trimethylamine to yield glycidyltrimethylammonium chloride and in the process, one molecule of CHPM would be converted to GMA. Suitable compounds may be selected from the group 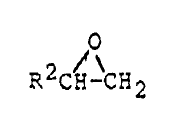
where R² selected from the group consisting of hydrogen, an alkyl or alkenyl group of 1 to 10 carbon atoms, an alkaryl or aralkyl group of 7 to 11 carbons, amine or trimethylammonium ion. Other suitable compounds include, but are not limited to, glycidyl acrylate, glycidyl methacrylate (GMA), allyl glycidyl ether, glycidyl trimethylammonium chloride, propylene oxide and epichlorohydin. The higher the ratio of the epoxy containing "catalyst" to CHPM, the faster the reaction proceeds. Preferred ratios range from 0.001:1.0 to 0.1:1.0. More preferably, the ratio is from 0.01:1.0 to 0.08:1.0.

Crystal purity is critical to the formation of linear high molecular weight polymers. In the reaction, impurities such as HPDMA and CPDMA are present due either to their presence in the CHPM or their formation during the course of the reaction with trimethylamine. They may result from the reaction of CHPM with residual methacrylic acid or from transesterifications. Other impurities which may be present include 1,3-bis(trimethylammonium)-2-hydroxypropane dichloride (DIQUAT); 2,3-dihydroxypropylmethacrylate; 2,3-dihydroxypropyltrimethylammonium chloride (DHPTAC) and methacrylic acid. These are not crosslinking agents and are not particularly detrimental to the formation of high molecular weight polymers even if present at levels of a few percent.

It has been reported that the rate of MAHTAC formation may be enhanced by the presence of quaternary ammonium salts, however, the presence of solid MAHTAC at the beginning of the reaction can increase the size of the crystals generated. Crystals with a size greater than about 40 µm have been found to be higher in purity (lower in CPDMA, HPDMA and residual starting material) and easier to separate from the reaction medium than smaller ones.

The following examples are included to illustrate the preparation of the compositions of the present invention, but are not to be considered limiting. Unless otherwise specified, all parts are parts by weight and all temperatures are expressed as degrees Celsius.

### Example 1

This example is directed to the synthesis of 3-chloro-2-hydroxypropylmethacrylate and its isomer.

To a 500 mL 3-necked, round bottom flask, equipped with a thermometer, air driven stirrer and reflux condenser, are charged 172.19 grams (2 moles) of methacrylic acid, 94.422 grams (2.1 moles) of epichlorohydrin, 0.88 grams of p-methoxyphenol (MEHQ) as a polymerisation inhibitor, and 8.1 grams (0.08 moles) of triethylamine as a catalyst. The reaction mixture is heated to 85°C for 5 hours. The yield is greater than 95% based on methacrylic acid. At the end of the reaction the following composition is measured by gas chromatography:

| Component | Weight Percent |
|---|---|
| CHPM & isomer | 88.41 |
| Epichlorohydrin | 0.90 |
| Methacrylic Acid | 0.73 |
| Dichloropropanol | 3.61 |
| GMA | 1.44 |
| HPDMA | 1.11 |
| MEHQ | 0.24 |
| Chloropropanediol | 0.25 |
| Triethylamine | 2.28 |
| DHPMA | 0.66 |
| CPDMA | 0.36 |

### Example 2

To a 500 mL, 3-necked, round bottom flask, equipped with a thermometer, air driven stirrer and reflux condenser, are charged 172.31 grams (2 moles) of methacrylic acid, 194.4 rams (2.1 moles) of epichlorohydrin, 0.86 grams of p-methoxyphenol (MEHQ) as a polymerization inhibitor, and 7.43 grams (0.04 moles) of benzyltrimethylammoniium chloride as a catalyst. The reaction mixture is heated to 85°C and a significant exotherm was noted as the temperature approached 85°C. The temperature is then maintained at 85°C for 5 hours. The yield is greater than 95%, based on methacrylic acid.

At the end of the reaction the following composition is, as measured by gas chromatography,:

| Component | Weight Percent |
|---|---|
| CHPM & isomer | 88.27 |
| Epichlorohydrin | 1.27 |
| Methacrylic Acid | 0.67 |
| Dichloropropanol | 3.97 |
| GMA | 0.68 |
| HPDMA | 1.22 |
| MEHQ | 0.24 |
| DHPMA | 0.95 |
| CPDMA | 0.18 |
| Catalyst | 0.95 |

### Example 3

The CHPM of Example 1 is purified by first extracting the saturated aqueous sodium bicarbonate to remove methacrylic acid followed by 2 % sulfuric acid and water. After vacuum distillation, a light yellow CHPM solution with greater than 97% purity is obtained.

The product has the following composition (by gas chromatography):

| Component | Weight Percent |
|---|---|
| CHPM & isomer | 97.31 |
| Epichlorohydrin | 0.24 |
| Dichloropropanol | 0.81 |
| GMA | 0.07 |
| HPDMA | 0.40 |
| MEHQ | 0.81 |
| DHPMA | 0.36 |

### Example 4

This example demonstrates the synthesis of crystalline MAHTAC from the unpurified CHPM of Example 1.

To a 500 mL Fisher Porter pressure bottle equipped with a magnetic stirrer are charged 150.02 grams (0.743 mole) of CHPM from Example 1, 155.72 grams of acetonitrile, 0.25 grams of MEHQ and 48.4 grams (0.819 mole) of trimethylamine. The bottle is sealed and heated in an oil bath at 65°C for 22 hours. The crystals began to precipitate after 45 minutes of reaction. The reaction mixture is filtered and dried through a Buchner funnel. The yield of MAHTAC is 93% of theory. Analysis of the crystals by liquid chromatography showed that they are 95.8% MAHTAC,

### Example 5

This example demonstrates the synthesis of crystalline MAHTAC from the unpurified CHPM OF Example 2.

To a 500 mL Fisher Porter pressure bottle equipped with a magnetic stirrer, are charged 150.03 grams (0.746 mole) of CHPM from Example 2, 154.05 grams of acetonitrile, 0.2 grams of MEHQ and 49.5 grams (0.837 mole) of trimethylamine. The bottle is sealed and heated in an oil bath at 65°C for 10 hours. The solution began to turn cloudy after 30 minutes of reaction. The reaction mixture is filtered and dried through a Buchner funnel. The yield of MAHTAC is 98.2% of theory. Analysis of the crystals by liquid chromatography showed they are 94.9% MAHTAC, 1.9% DHPTAC AND 3.2% DIQUAT. The melting point is 180-182°C.

### Example 6

To a 500 mL Fisher Porter pressure bottle equipped with a magnetic stirrer are charged 100.12 grams (0.5454 mole) of purified CHPM from Example 3, 100.12 grams of acetonitrile, 0.22 grams of MEHQ and 54.6 grams (0.924 mole) of trimethylamine. The bottle is sealed and heated in an oil bath at 65°C for 35 hours. The solution began to turn cloudy after 30 minutes of reaction. The reaction mixture is filtered and dried through a Buchner funnel. The yield of MAHTAC is 93.3% of theory. Analysis of the crystals by liquid chromatography showed they are 98% MAHTAC, 2% DHPTAC and essentially no DIQUAT. The melting point was 181-183°C.

### Example 7 [Comparative]

This example demonstrates that the process of this invention differs significantly from that of British Patent 1,112,912. In Example 1 of '912, the manufacture of 3-methacryloyloxy-2-hydroxypropyltrimethylammonium chloride from the reaction of potassium methacrylate (PMA) and 3-chloro-2-hydroxypropyltrimethylammonium chloride (CHPTAC) is disclosed. The MAHTAC is synthesized in a 500 mL 3-neck flask fitted with a thermometer, stirrer and a reflux condenser. Into the flask are placed 18.8 parts CHPTAC, 12.4 parts of PMA, 200 mL of acetonitrile and 0.01 parts of MEHQ as a polymerization inhibitor. The reaction mixture is heated under reflux for 2.5 hours and then allowed to stand at room temperature overnight. The reaction mixture is filtered to remove 7.72 parts of potassium chloride. The acetonitrile is distilled from the filtrate under reduced pressure, leaving as a residue 22.0 parts (93% of theory) of MAHTAC product. The crude MAHTAC product is this purified by recrystallization from a 1:1 mixture of isopropanol and ethyl acetate.

The experiment essentially as described is repeated but with very different results. Since no weight unit is given for the reacting components, three experiments are done with one part taken as 1.0 gram, 0.5 gram and 0.2 gram. The reaction conditions are descried in Table I below.

**Table I**

| Experiment Number | Reaction Mixture | Weight (g) | Moles | Reaction Hrs. | Yield % |
|---|---|---|---|---|---|
| 1 | PMA | 12.42 | 0.10 | 2.5 | 51.1 |
| | CHPTAC | 18.91 | 0.10 | | |
| | MEHQ | 0.01 | | | |
| | CH₃CN | 155.2 | | | |
| 2 | PMA | 6.28 | 0.05 | 2.5 | 44.8 |
| | CHPTAC | 9.48 | 0.05 | | |
| | MEHQ | 0.01 | | | |
| | CH₃CN | 155.10 | | | |
| 3 | PMA | 2.47 | 0.02 | 2.5 | 47.4 |
| | CHPTAC | 3.74 | 0.02 | | |
| | MEHQ | 0.01 | | | |
| | CH₃CN | 155.10 | | | |

All reactions are heterogeneous and the purity of the MAHTAC after recrystallization is 49.1, 58.2, and 47.9 for experiments 1,2 and 3 respectively.

Examples 8 through 11 demonstrate that the rate of formation of MAHTAC can be greatly enhanced by the addition of an epoxy containing compound to the reaction mixture. The experimental results are presented in Table II below.

### Example 8

This example shows the rate of MAHTAC formation when no epoxy compounds are present in the reaction mixture. As shown in Table II, only 33.3% of the CHPM is converted to MAHTAC in three hours at 65°C when no glycidylmethacrylate was present. The product purity is 97.9%.

### Example 9

When 1.09% GMA is present in the reaction mixture, 69.1% of the CHPM formed MAHTAC in three hours. This is a significant rate enhancement over Example 8. The product purity is 98.6%.

### Example 10

When 3.27% GMA is present in the reaction mixture, 98.6% of the CHPM is converted to MAHTAC in three hours. This is a further improvement over Example 9. The MAHTAC purity is 97.9%.

### Example 11

This example shows that the rate enhancement is also seen at 45°C. A three hour CHPM conversion of 54.3% is found and the product is 98.3% pure.

**Table II**

| Reactants(g) | Ex.8 | Ex.9 | Ex.10 | Ex.11 |
|---|---|---|---|---|
| CHPM | 100.12 | 100.13 | 100.14 | 100.15 |
| TMA | 35.9 | 35.3 | 35.7 | 35.7 |
| CH₃CN | 100.12 | 99.05 | 96.83 | 96.81 |
| MEHQ | 0.22 | 0.22 | 0.23 | 0.22 |
| GMA | 0.0 | 1.09 | 3.27 | 3.28 |

| Conditions | | | | |
|---|---|---|---|---|
| Temperature(°C) | 65 | 65 | 65 | 45 |
| Conversion at 3 Hrs. | 33.27 | 69.08 | 98.62 | 54.29 |
| Reaction time(Hrs) | 25 | 7 | 3 | 7 |

| Product | | | | |
|---|---|---|---|---|
| Crystal Wt (g) | 93.04 | 106.4 | 121.2 | 124.4 |
| Yield(%) | 87.23 | 91.7 | 98.32 | 97.87 |
| MAHTAC purity(%) | 97.94 | 98.61 | 97.93 | 98.3 |

### Example 12

Table III below shows that the same rate enhancement effect may be achieved by substituting another epoxide for GMA. In this example, equivalent molar amounts of GMA and glycidyl allyl ether (GAE) are used in two essentially identical reactions. The results are very similar, and many other epoxides may be suitable for enhancing the reaction rate.

**Table III**

| Reactants(g) | Ex.12 | Ex.13 |
|---|---|---|
| CHPM | 100.15 | 100.10 |
| TMA | 36.4 | 38.7 |
| CH₃CN | 96.84 | 97.41 |
| MEHQ | 0.21 | 0.21 |
| GMA | 3.25 | 0.0 |
| GAE | 0.0 | 2.69 |
| Total Wt. | 236.85 | 239.11 |

| Conditions | | |
|---|---|---|
| Temperature(°C) | 65 | 65 |
| Conversion at 3 Hrs. | 97.09 | 98.27 |
| Reaction time(Hrs.) | 3.5 | 3.5 |

| Product | | |
|---|---|---|
| Crystal Wt(g) | 114.16 | 120.8 |
| Yield(%) | 88.44 | 93.57 |
| MAHTAC purity(%) | 95.23 | 96.77 |

### Example 13

This Example is directed to the preparation of 3-chloro-2-hydroxypropylmethacrylate (CHPM).

To a 500 mL. 3-necked round bottomed flask, equipped with a thermometer, agitator and reflux condenser, are charged 172.31 grams (2 moles) of methacrylic acid (MAA), 194.40 grams (2.1 moles) of epichlorohydrin (EPI), 0.86 gram (0.5 weight % of MAA) of p-methoxyphenol as polymerization inhibitor and 9.52 grams (0.04 mole) of 3-methacryloyloxy-2-hydroxypropyltrimethylammonium chloride as catalyst. The reaction mixture is heated to 85°C for 7 hours. A slight exotherm is observed in the first hour of the reaction. The yield of the CHPM is 95% based on methacrylic acid and the purity of the CHPM was about 88.27%. Gas chromatographic analysis shows that the CHPM solution has the following composition (weight percent): CHPM 88.27%, EPI 1.27%, MAA 0.67% DCP 3.97%, GMA 0.68%, HPDMA 1.22%, MEHQ 0.24%, CPDMA 0.18%, DHPMA 0.95%, and catalyst 2.55%.

The crude CHPM solution is then extracted with a saturated aqueous NaHCO₃ until the disappearance of acid reaction (to remove residual methacrylic acid), then with a 2% sulfuric acid, and after that with water. After vacuum distillation to remove residual epichlorohydrin and water, a light yellow CHPM solution with greater than 97% purity is obtained.

### EXAMPLES 14-16

Examples 14-16 are directed to the preparation of 3-chloro-2-hydroxypropylmethacrylate (CHPM).

The procedure of Example 13 is repeated under the conditions shown in Table IV below. Each reaction is conducted in the presence of 0.43 gram (0.5 weight % of MAA) of p-methoxyphenol. In order to minimize exothermic reaction. the reaction temperature at the first hour is kept at 65°C and then gradually raised to 85%C. The yield of the CHPM is calculated. The reaction rate constant (K) in each Example is the slope of the plot of -ln ([MAA]/[MAAₒ]) vs reaction time where [MAA] is the amount of methacrylic acid after a given reaction time, and [MAAₒ] is the initial concentration of methacrylic acid. All values are in mole percent. The results are in Table IV below.

**Table IV**

| EXP | [EPI]/[MAA] | [MAHTAC]/[MAA] | Temp. °C | Time (Hrs) | K | Percent Yield |
|---|---|---|---|---|---|---|
| 2 | 1.05 | 0.02 | 75 | 7 | 0.3179 | 97 |
| 3 | 1.05 | 0.03 | 75 | 6 | 0.4581 | 99 |
| 4 | 1.05 | 0.04 | 75 | 5 | 0.5287 | 97 |

Plotting [MAHTAC]/[MAA] against K indicates that K in the formation of CHPM is a linear function of tho MAHTAC catalyst concentration.

### EXAMPLE 17

This Example is directed to the preparation of 3-methacryloyloxy-2-hydroxypropyltrimethylammonium chloride.

To a 500 mL. Fisher & Porter pressure bottle equipped with a magnetic stirrer are charged 151 grams (0.746 mole) of unpurified CHPM solution from Example 1, 154.05 grams of acetonitrile, 0.12 gram of p-methoxyphenol, and 49.5 grams (0.837 mole) of trimethylamine. The bottle is sealed and heated in an oil bath at 65°C for 10 hours. The reaction mixture starts to turn cloudy after 30 minutes of stirring. A white crystalline MAHTAC monomer is obtained with a 98% yield. The average particle size of MAHTAC monomer product is 30 to 40 µm. Liquid chromatographic analysis shows that the white crystal is a composition of 94.9% of MAHTAC monomer, 3% of 2-hydroxypropane-1,3-bis(trimethylammonium) dichloride (DIQUAT), and less than 1.9% of 2,3-dihydroxypropyltrimethylammonium chloride (DHPTAC). Other impurities determined by liquid chromatography of ether extracts are MAA 12 ppm., GMA 18 ppm., CHPM 350 ppm., DHPMA 87 ppm., and HPDMA 2244 ppm.

### Example 18

This Example illustrates the seeding effect of the MAHTAC monomer in the second step of the MAHTAC monomer preparation.

A procedure sirilar to that of Example 17 is followed. During the course of quaternization, a small amount of fine MAHTAC crystals in an amount of 5 weight % of CHPM is added into the batch liquor (supersaturated solution). It is found that the average particle size of crystals increases to 200 µm and impurity levels dropped by a factor of four.

### Example 19

This example demonstrates the high purity of MAHTAC made by the process of the present invention. The following two experiments were carried out in an agitated, 7-liter, stainless steel, jacketed, pressure reactor, fitted with temperature and pressure instrumentation.

### Experiment A

To the above reactor are added 400 grams of MAHTAC, 1500 grams of CHPM, 2600 grams of acetonitrile and 500 grams of trimethylamine. The reactor is heated to 50°C and allowed to react for 19.5 hours. The contents of the reactor are then added to a 12 inch diameter, perforate basket centrifuge containing a teflon filter cloth and separated from the mother liquor (ML) at 1960 rpm. They are then washed with 330 grams of acetonitrile at low speed followed by spinning again at 1960 rpm. The crystals are viewed under the microscope and found to have a mean size of 57.8 µm. The impurity levels for both the mother liquor and the washed crystals are listed in Table V below.

### Experiment B

To the above reactor are added 120 grams of glycidyl methacrylate, 1500 grams CHPM, 2600 grams acetonitrile and 500 grams TMA. The reactor is heated to 50°C for 5 hours. Following the procedure in the above experiment the reactor contents are centrifuged and washed. Microscopic examination of the crystals showed that they had a mean particle size of 16.5 µm. The impurity levels are listed in Table V below.

**Table V**

| | Experiment A (Large Crystal) | | Experiment B (Small Crystal) | |
|---|---|---|---|---|
| Impurity | ML(%) | (ppm) | ML(%) | (ppm) |
| DHPMA | 0.21 | 2.5 | 0.27 | 5.0 |
| MAA | 0.38 | 0.5 | 0.62 | 1.5 |
| CHPM | 1.6 | 28 | 1.2 | 95 |
| GMA | 1.2 | ND | 4.6 | 44 |
| HPDMA | 2.3 | 22 | 1.2 | 50 |
| CPDMA | 4.9 | 35 | 4.8 | 130 |
| GTMA | 0.76 | 12 | 0.78 | 35 |

Table V shows the levels of the impurities found in the crystal mother liquor and in the washed crystal for the two experiments of Example 19. These experiments shown that a significant reduction (up to 73%) in crosslinking impurities can be achieved by increasing the crystal size from 16.5 to 57.8 µm.

## Claims

1. A process for preparing a mixture of a 3-(R)acryloyloxy-2-hydroxypropyltrialkylammonium halide with its 2-(R)acryloyloxy-3-hydroxypropyltrialkylammonium halide isomer, characterised by reacting the halohydroxypropyl ester of an acid of the formula CH₂=C(R)COOH wherein R is hydrogen or alkyl of 1 to 4 carbon atoms with from 0.5 to 2.0 moles, per mole of halohydroxy propyl ester, of a trialkylamine at a temperature of 25 to 90°C in from 35 to 85 weight % of a polar aprotic liquid which is a non-solvent for said 3-(R)acryloyloxy-2-hydroxypropyltrialkylammonium halide and its isomer.

2. A process according to Claim 1 characterised in that the molar ratio of hydroxypropyl ester to trialkylamine is from 1.0:1.0 to 1.0:2.0.

3. A process according to Claim 1 or 2 characterised in that the temperature is from 50 to 85°C.

4. A process according to any one of Claims 1 to 3 characterised in that the non-solvent is selected from acetonitrile; acetone; methyl ethyl ketone; tetrahydrofuran; ethylacetate and 1,1,2-trichloroethene.

5. A process according to Claim 4 characterised in that the non-solvent is acetonitrile.

6. A process according to any one of Claims 1 to 5 characterised in that reaction occurs in the presence of a polymerization inhibitor.

7. A process according to any one of Claims 1 to 5 characterised in that a catalyst is used and the molar ratio of chlorohydroxypropyl ester to catalyst is from 1.0:0.001 to 1.0:0.1.

8. A process according to Claim 7 characterised in that the catalyst is present in an amount from 1.0:0.01 to 1.0:0.08.

9. A process according to Claim 7 or 8 characterised in that the catalyst contains an epoxy functional group.

10. A process according to Claim 9 characterised in that the catalyst is an epoxy compound having the formula: 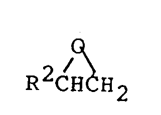 wherein R² is hydrogen alkyl of 1 to 10 carbon atoms, alkenyl of 1 to 10 carbon atoms, alkaryl of 7 to 11 carbon atoms, aralkyl of 7 to 11 carbon atoms, amine and trimethylammonium ion.

11. A process according to Claim 10 characterised in that the catalyst is glycidyl methacrylate, alkyl glycidyl ether, glycidyl acrylate, glycidyl trimethylammonium chloride or propylene oxide.

12. A process according to Claim 7 or 8 characterised in that the catalyst is 3-(R)acryloyloxy-2-hydroxypropyltrialkylammonium chloride and its isomer.

13. A process according to Claim 12 characterised in that the catalyst is 3-methacryloyloxy-2-hydroxypropyltrimethylammonium chloride and its isomer.

14. A process according to any one of Claims 1 to 13 characterised in that the halohydroxypropyl ester is prepared by reacting methacrylic acid and epichlorohydrin in the presence of a mixture of 3-methacryloyloxy-2-hydroxypropyltrimethylammonium chloride monomer and its isomer, 2-methacryloyloxy-3-hydroxypropyltrimethylammonium chloride monomer as catalyst at a temperature and for a time sufficient to form said ester.

15. A process according to Claim 14 characterized in that the molar ratio of methacrylic acid to epichlorohydrin is from 0.5:1.0 to 1.0:0.5.

16. A process according to Claim 14 characterised in that the molar ratio of the mixture of 3-methacryloyloxy-2-hydroxypropyltrimethylammonium chloride monomer and its isomer to methacrylic acid is from 0.01:1.0 to 0.2:1.0.

17. A process according to any one of Claims 14 to 16 characterised in that the reaction of methacrylic acid and epichlorohydrin is carried out at a temperature of 25 to 100°C.

18. A process according to any one of Claims 14 to 17 characterised in that the reaction of methacrylic acid and epichlorohydrin is carried out in the presence of a polymerization inhibitor.

19. A process according to any one of Claims 14 to 18 characterised in that the reaction of methacrylic acid and epichlorohydrin is carried out in the presence of a non-solvent.

## Patentansprüche

1. Verfahren zur Herstellung eines Gemisches aus einem 3-(R)Acryloyloxy-2-hydroxypropyltrialkylammoniumhalogenid mit dessen 2-(R)Acryloyloxy-3-hydroxypropyltrialkylammoniumhalogenid-Isomers,
**gekennzeichnet** durch
Umsetzen des Halohydroxypropylesters einer Säure der Formel CH₂=C(R)COOH, worin R für Wasserstoff oder Alkyl mit 1 bis 4 Kohlenstoffatomen steht, mit 0,5 bis 2,0 Mol, pro Mol an Halohydroxypropylester, eines Trialkylamins bei einer Temperatur von 25 bis 90 °C in 35 bis 85 Gew.-% einer polaren aprotischen Flüssigkeit, bei der es sich um ein Nicht-Lösungsmittel für das 3-(R)Acryloyloxy-2-hydroxypropyltrialkylammoniumhalogenid und dessen Isomer handelt.

2. Verfahren nach Anspruch 1,
dadurch **gekennzeichnet**,
daß das Molverhältnis von Hydroxypropylester zu Trialkylamin 1,0:1,0 bis 1,0:2,0 beträgt.

3. Verfahren nach Anspruch 1 oder 2,
dadurch **gekennzeichnet**,
daß die Temperatur 50 bis 85 °C beträgt.

4. Verfahren nach einem der Ansprüche 1 bis 3,
dadurch **gekennzeichnet**,
daß das Nicht-Lösungsmittel ausgewählt ist unter Acetonitril, Aceton, Methylethylketon, Tetrahydrofuran, Ethylacetat und 1,1,2-Trichlorethen.

5. Verfahren nach Anspruch 4,
dadurch **gekennzeichnet**,
daß es sich bei dem Nicht-Lösungsmittel um Acetonitril handelt.

6. Verfahren nach einem der Ansprüche 1 bis 5,
dadurch **gekennzeichnet**,
daß die Umsetzung in Anwesenheit eines Polymerisationsinhibitors stattfindet.

7. Verfahren nach einem der Ansprüche 1 bis 5,
dadurch **gekennzeichnet**,
daß ein Katalysator eingesetzt wird und das Molverhältnis von Chlorhydroxypropylester zu Katalysator 1,0:0,001 bis 1,0:0,1 beträgt.

8. Verfahren nach Anspruch 7,
dadurch **gekennzeichnet**,
daß der Katalysator in einer Menge von 1,0:0,01 bis 1,0:0,08 vorhanden ist.

9. Verfahren nach Anspruch 7 oder 8,
dadurch **gekennzeichnet**,
daß der Katalysator eine funktionelle Expoxygruppe enthält.

10. Verfahren nach Anspruch 9,
dadurch **gekennzeichnet**,
daß es sich bei dem Katalysator um eine Expoxyverbindung der folgenden Formel: 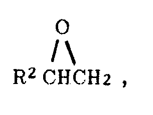 worin R² für Wasserstoff, Alkyl mit 1 bis 10 Kohlenstoffatomen, Alkenyl mit 1 bis 10 Kohlenstoffatomen, Alkaryl mit 7 bis 11 Kohlenstoffatomen, Aralkyl mit 7 bis 11 Kohlenstoffatomen, Amin und Trimethylammoniumion steht, handelt.

11. Verfahren nach Anspruch 10,
dadurch **gekennzeichnet**,
daß es sich bei dem Katalysator um Glycidylmethacrylat, Alkylglycidylether, Glycidylacrylat, Glycidyltrimethylammoniumchlorid oder Propylenoxid handelt.

12. Verfahren nach Anspruch 7 oder 8,
dadurch **gekennzeichnet**,
daß es sich bei dem Katalysator um 3-(R)Acryloyloxy-2-hydroxypropyltrialkylammoniumchlorid und dessen Isomer handelt.

13. Verfahren nach Anspruch 12,
dadurch **gekennzeichnet**, daß es sich bei dem Katalysator um 3-Methacryloyloxy-2-hydroxypropyltrimethylammoniumchlorid und dessen Isomer handelt.

14. Verfahren nach einem der Ansprüche 1 bis 13,
dadurch **gekennzeichnet**,
daß der Halohydroxypropylester hergestellt wird, indem Methacrylsäure und Epichlorhydrin in Anwesenheit eines Gemisches aus 3-Methacryloyloxy-2-hydroxypropyltrimethylammoniumchlorid-Monomer und dessen Isomer, 2-Methacryloyloxy-3-hydroxypropyltrimethylammoniumchlorid-Monomer als Katalysator bei einer solchen Temperatur und während einer solchen Zeitspanne umgesetzt werden, daß dieser Ester gebildet wird.

15. Verfahren nach Anspruch 14,
dadurch **gekennzeichnet**,
daß das Molverhältnis von Methacrylsäure zu Epichlorhydrin 0,5:1,0 bis 1,0:0,5 beträgt.

16. Verfahren nach Anspruch 14,
dadurch **gekennzeichnet,**
daß das Molverhältnis des Gemisches aus 3-Methacryloyloxy-2-hydroxypropyltrimethylammoniumchlorid-Monomer und dessen Isomer zur Methacrylsäure 0,01:1,0 bis 0,2:1,0 beträgt.

17. Verfahren nach einein der Ansprüche 14 bis 16,
dadurch **gekennzeichnet**,
daß die Umsetzung von Methacrylsäure und Epichlorhydrin bei einer Temperatur von 25 bis 100 °C durchgeführt wird.

18. Verfahren nach einem der Ansprüche 14 bis 17,
dadurch **gekennzeichnet**,
daß die Umsetzung von Methacrylsäure und Epichlorhydrin in Anwesenheit eines Polymerisationsinhibitors durchgeführt wird.

19. Verfahren nach einem der Ansprüche 14 bis 18,
dadurch **gekennzeichnet**,
daß die Umsetzung von Methacrylsäure und Epichlorhydrin in Anwesenheit eines Nicht-Lösungsmittels durchgeführt wird.

## Revendications

1. Un procédé de préparation d'un mélange d'halogénure de 3-(R)-acryloyloxy-2-hydroxypropyltrialkylammonium avec son isomère correspondant à l'halogénure de 2-(R)-acryloyloxy-3-hydroxypropyltrialkylammonium, caractérisé par la réaction de l'ester halohydroxypropylique d'un acide de formule:
CH₂=C(R)COOH
dans laquelle:
R représente un atome d'hydrogène ou un radical alkyle comportant de 1 à 4 atomes de carbone,
avec entre 0,5 et 2,0 moles par mole d'ester halohydroxypropylique d'une trialkylamine, à une température comprise entre 25°C et 90°C, dans 35 à 85% en poids d'un liquide aprotique polaire qui ne correspond pas à un solvant pour le dihalogénure de 3-(R)-acryloyloxy-2-hydroxypropyltrialkylammonium et pour son isomère.

2. Un procédé selon la revendication 1, caractérisé en ce que le rapport molaire ester hydroxypropylique/trialkylamine est compris entre 1,0/1,0 et 1,0/2,0.

3. Un procédé selon la revendication 1 ou 2, caractérisé en ce que la température est comprise entre 50°C et 85°C.

4. Un procédé selon l'une quelconque des revendications 1 à 3, caractérisé en ce que le liquide ne correspondant pas à un solvant est sélectionné parmi: acétonitrile, acétone, méthyléthylcétone, tétrahydrofuranne, acétate d'éthyle et 1,1,2-trichloroéthène.

5. Un procédé selon la revendication 4, caractérisé en ce que le liquide ne correspondant pas à un solvant est l'acétonitrile.

6. Un procédé selon l'une quelconque des revendications 1 à 5, caractérisé en ce que la réaction est réalisée en présence d'un inhibiteur de polymérisation.

7. Un procédé selon l'une quelconque des revendications 1 à 5, caractérisé en ce qu'un catalyseur est utilisé et en ce que le rapport molaire esterchloropydroxypropylique/catalyseur est compris entre 1,0/0,001 et 1,0/0,1.

8. Un procédé selon la revendication 7, caractérisé en ce que le catalyseur est présent en une quantité comprise entre 1,0/0,01 et 1,0/0,08.

9. Un procédé selon la revendication 7 ou 8, caractérisé en ce que le catalyseur contient un groupe fonctionnel époxy.

10. Un procédé selon la revendication 9, caractérisé en ce que le catalyseur est un dérivé époxy représenté par la formule: 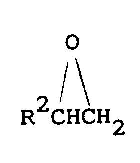 dans laquelle:
R² représente un atome d'hydrogène, un radical alkyle comportant de 1 à 10 atomes de carbone, un radical alcényle comportant de 1 à 10 atomes de carbone, un radical alcaryle comportant de 7 à 11 atomes de carbone, une amine ou un ion triméthylammonium.

11. Un procédé selon la revendication 10, caractérisé en ce que le catalyseur correspond à: méthacrylate de glycidyle, éther d'alkylglycidyle, acrylate de glycidyle, chlorure de glycidyltrialkylammonium ou oxyde de propylène.

12. Un procédé selon la revendication 7 ou 8, caractérisé en ce que le catalyseur correspond au chlorure de 3-(R)-acryloyloxy-2-hydroxypropyltrialkylammonium et à son isomère.

13. Un procédé selon la revendication 12, caractérisé en ce que le catalyseur correspond au chlorure de 3-méthacryloyloxy-2-hydroxypropyltriméthylammonium et à son isomère.

14. Un procédé selon l'une quelconque des revendications 1 à 13, caractérisé en ce que l'ester d'halohydroxypropyle est préparé par réaction de l'acide méthacrylique et de l'épichlorhydrine en présence d'un mélange de monomère de chlorure de 3-méthacryloyloxy-2-hydroxypropyltriméthylammonium et de son isomère: le monomère de chlorure de 2-méthacryloyloxy-3-hydroxypropyltriméthylammonium en tant que catalyseur à une température et pendant une période de temps suffisantes pour former ledit ester.

15. Un procédé selon la revendication 14, caractérisé en ce que le rapport molaire acide méthacrylique/épichlorhydrine est compris entre 0,5/1,0 et 1,0/0,5.

16. Un procédé selon la revendication 14, caractérisé en ce que le rapport molaire mélange de monomère de chlorure de 3-méthacryloyloxy-2-hydroxypropyltriméthylammonium et de son isomère/acide méthacrylique est compris entre 0,01/1,0 et 0,2/1,0.

17. Un procédé selon l'une quelconque des revendications 14 à 16, caractérisé en ce que la réaction de l'acide méthacrylique et de l'épichlorhydrine est mise en oeuvre en présence d'un inhibiteur de polymérisation.

18. Un procédé selon l'une quelconque des revendications 14 à 17, caractérisé en ce que la réaction de l'acide méthacrylique et de l'épichlorhydrine est mise en oeuvre en présence d'un inhibiteur de polymérisation.

19. Un procédé selon l'une quelconque des revendications 14 à 18, caractérisé en ce que la réaction de l'acide méthacrylique et de l'épichlorhydrine est mise en oeuvre en présence d'un liquide non solvant.
